# EUROPEAN PATENT APPLICATION

(11) **EP 4 649 992 A2**
(43) Date of publication of application: **19.11.2025**
(21) Application number: 25199751.6
(22) Date of filing: 30.07.2021
(51) Int. Cl.: A61N 1/05

(54) **SYSTEM FOR INSERTION OF A COCHLEAR IMPLANT**

(30) Priority: 30.07.2020 US 202063058508 P
(62) Divisional of application: 21752228.3
(71) Applicant: CAScination AG, 3008 Bern (CH)
(72) Inventor: Weber, Stefan, 3008 Bern (CH); Matulic, Marco, 3008 Berlin (CH)
(74) Representative: Schulz Junghans Patentanwälte PartGmbB

(57) **Abstract**

A system and method for surgical implantation of cochlear implants that allows for the assessment of cochlear implant insertion progress during the surgical procedure through insertion planning, electrode selection, monitoring insertion progress, and confirming implant placement is provided. The inventive system and methods allow for the determination of a desired insertion state and progress plan for the selected cochlear implant and subsequently allow for the monitoring of insertion progress The provided system and methods allow for the use of imaging data, implant data, patient data and electrophysiological data in determining the desired insertion state and progress plan and for selecting the implant, and various imaging and tracking methodologies in monitoring insertion progress and confirming that the implant has been properly placed. Provided are an inventive system and methods that ensure quality cochlear implant insertion through insertion planning, proper electrode selection, insertion monitoring and confirmation of implant placement.

## Description

### FIELD OF THE INVENTION

The present invention relates to the field of cochlear implantation. Specifically, the invention pertains to a system and method for surgical insertion of cochlear implants that allows for the assessment of cochlear implant insertion progress during the surgical procedure through insertion planning, monitoring insertion progress, and confirming implant placement. The inventive system and methods allow for the determination of a desired insertion state and progress plan for the cochlear implant, optimal implant selection for the patient, and subsequently allow for the monitoring of insertion progress Specifically, the inventive system and methods allow for the use of imaging data, implant data, patient data and electrophysiological data in determining the desired insertion state and progress plan and various imaging and tracking methodologies in monitoring insertion progress and confirming that the implant has been properly placed. Most specifically, the inventive system and methods relate to quality cochlear implant insertion through insertion planning, implant selection, insertion monitoring and confirmation of implant placement.

### BACKGROUND OF THE INVENTION

Cochlear implant surgery ("CI") consists of three main steps. First, a mastoidectomy is performed, whereby the mastoid bone is opened, providing access to the middle ear space. Next, the surgeon navigates the middle ear space, taking care to avoid key structures such as the facial nerve and chorda tympani. Once entered into the middle ear cavity, the surgeon creates an opening either into the round window membrane of the cochlea (round window approach) or into the cochlea wall (cochleostomy). Finally, the electrode array is inserted into the cochlea. Each step of CI carries risks and challenges, including the possibility of damage to nerves lying in the lateral skull base, incorrect or incomplete insertion of the electrode array, damaging of intracochlear structures or sub-optimal choice of cochlear implant for the individual patient.

Several elements of robotic cochlear implantation, such as minimally invasive mastoidectomy have been demonstrated using computer assisted and template-based approaches (Labadie), or using robotic technology (Weber et al., Science Robotics 2017). In addition, automatic / mechatronic inner ear access has been demonstrated (Peter Brett et al., 2014). Moreover using mechatronic approaches to deliver CI electrodes with defined insertion speeds have been investigated.

Computer assisted planning of CI procedures has been described utilizing medical images, to identify relevant anatomy (e.g., Cochlear) to plan for access routes into the cochlea. To date commercial tools such as OTOPLAN are used to assist in an optimal electrode choice with respect to available imaging and audiological data of a given patient. (selecting the desired length of the CI electrode with respect to anatomy).

Specifically, the approach involves drilling a small hole directly in the mastoid surface with the aim of producing a small diameter tunnel (in the range of 1.5-2mm in diameter) providing direct access to a target site on the cochlea. This surgical approach traverses the facial recess and therefore the resultant tunnel passes close to the facial nerve, the chorda tympani, the auditory canal, and the ossicles. Accordingly, the biggest challenge associated with performing an RCI approach to cochlear implantation is avoiding damage to those structures, particularly the facial nerve and chorda tympani. While current surgical practice has accepted the sacrifice of the chorda tympani in a percentage of cases, it is highly undesirable and, in any event, damage to, or physical destruction of, the facial nerve is an unacceptable outcome of CI surgery.

Due to the challenges associated with the RCI approach , various strategies for increasing safety and reducing the incidence of damage to the facial nerve and other structures have been investigated by the current inventors and other groups. None of the known strategies, standing on its own, provides the necessary safety margin for successful RCI procedures. The goal of quality cochlear implant insertion has not yet been achieved in the field, where quality insertion can be defined as the ability to place cochlear implants safely and accurately - without damage to surrounding structures while achieving a placement that truly enhances the patient's hearing to a worthwhile extent. In this regard, quality cochlear implantation involves planning of both surgical approach and optimal implant choice, monitoring of the surgical process, and confirmation that the desired surgical outcome has been reached.

RCI has not achieved anywhere near universal adoption due to factors such as cost, relative infancy of the underlying technology, and lack of a quality approach to robotic insertion. Accordingly, current practice still involves manual surgical insertion that is inherently destructive. Inserting a cochlear implant is actually beyond the limits of human dexterity and so approximately 50% of cochlear implant patients do not have the hearing performance they need after surgery. This is generally because the insertion process is so rough and destructive with respect to the inner ear structures, which can often result in destruction of any residual hearing the patient has. Accordingly, ordinary CI surgery is only cleared for deaf patients. Because of this dynamic, along with cost issues, implementation rates of cochlear implants in general are as low as 5%. As a result, in a worldwide population of 15 million with hearing problems that may be addressed by cochlear implants, perhaps only 70,000 actually receive implants.

As a result, there is a strong need in the field for a safer, results-driven approach to CI surgery, one that can likely only be achieved through quality, minimally invasive placement of cochlear implants.

Various incomplete attempts to improve RCI have been previously disclosed. In WO2019/157004, the inventors describe a tracking approach used to navigate/localise a cochlear implant using electromagnetic and/or electropotential tracking. US2018/0050196 describes a remote-controlled arm insertion of cochlear implants based at least in part on ECoG data. And US8594799 provides a limited disclosure of the robotic use case in a method of cochlear implant insertion primarily based on force sensing as a method of placement control.

While various safety measures have been described in the available literature, a significant drawback is that none of the current approaches sufficiently combine planning and monitoring modalities to arrive at a quality approach to cochlear implantation. For example, some known methods use tracking approaches for guiding insertion and/or confirming placement but do not coherently plan the implantation procedure in advance. Other approaches use electrophysiological data or force sensing to guide mechanical insertion of an implant, but do not determine a desired insertion pathway in advance and do not select cochlear implants accordingly. None of the known approaches combine surgical planning and/or monitoring with a process for selecting an optimal cochlear implant for the particular patient using preoperatively acquired patient data and a known database of population data.

Keeping these drawbacks in mind, the current inventors have realized that a consolidated approach involving insertion planning, implant selection, monitoring of insertion progress and confirming implant placement results in quality, reliable, accurate and safe placement of cochlear implants in a way not previously available.

### SUMMARY OF THE INVENTION

These aims and other advantages are achieved by a new system and method for the quality insertion of cochlear implants by minimally invasive or robotic means. The inventive system and methods allow for the determination of a desired insertion progress plan using available medical image data, electrophysiological data, genetic data, audiological and other clinical data. Insertion progress can be monitored using intraoperative imaging data, electrophysiological data, data on the position of tools, impedance data, implant properties and the interaction of the implant with various tissue types. Confirmation of desired implant placement can be achieved through imaging data, electrophysiological data, implant properties, implant interaction with tissue and implant performance.

Thus, according to an embodiment of the current invention, multiple, diverse data points are combined to develop a desired cochlear implant insertion state and insertion progress plan. This can include specific patient data, comprising physiological, audiological, image, genetic and chemical data. Population level data can also be incorporated to set general parameters for shape and size of the cochlea and tissue characteristics of various ear structures. Planning desired insertion state and progress can also incorporate the use of electrophysiological data.

In an embodiment, planning data is combined to determine insertion parameters, such as depth, speed, direction, orientation, speed profile and orientation profile. Data is also combined to determine desired insertion progress. So for example, desired implant position, orientation, bending and shape are assessed to arrive at a desired insertion state measured across position and time points.

In an alternative embodiment, planning data may be used in the selection of an optimal cochlear implant for the specific patient based upon patient data and its comparison to historical or average data.

According to an alternative embodiment, medical image data, data on the position of tools, electrophysiological data and properties of the implant and its interaction with tissue types are used to monitor insertion progress and compare measured insertion progress to the insertion plan. This comparison may be used to create a feedback loop where insertion trajectory, speed, orientation and other parameters can be altered to align insertion progress with the insertion plan.

According to an alternative embodiment, medical imaging data, electrophysiological data, implant properties, interaction of the implant with the cochlear tissue and implant performance may be used to confirm quality, accurate and safe implant placement.

In an alternative embodiment, planning data, monitoring data and confirmation of implant placement are combined in a system and method for quality insertion of cochlear implants.

In an alternative embodiment, medical image data, electrophysiological data, genetic data, audiological and other clinical data is input into an algorithm running on a computer that produces accordingly a desired insertion state and desired insertion progress plan. The algorithm may optionally test implant specific parameters against the desired insertion progress plan and desired insertion state, along with patient-specific data to choose an optimal cochlear implant for the specific patient. Similarly, the algorithm may work iteratively to arrive at an optimized set of insertion state and progress parameters for the specific implant and patient.

The inventive system and methods of the present invention may optimally be used in a minimally invasive approach to cochlear implantation. The inventors' combination of data sources and combination of planning, monitoring and confirmation of placement with minimally invasive techniques results in the best quality result for the patient. Optionally, the minimally invasive approach to quality implant placement may comprise robotic cochlear implantation guided by the planning, monitoring and confirmation approaches taught by the present inventors.

These and other embodiments of the inventive system and method are described in more detail below with reference to the attached figures.

### BRIEF DESCRIPTION OF THE FIGURES

- Figure 1 is a schematic of a method for insertion of a cochlear implant involving preoperative planning and intraoperative monitoring and confirmation, according to an embodiment of the present invention.
- Figure 2 is a schematic of a method of electrode selection involving the application of patient and population data to a database of available electrodes and generating a resulting score, according to an embodiment of the present invention.
- Figure 3 provides a view of a system for insertion of a cochlear implant incorporating a computerized planning tool and a robotic insertion tool, according to an embodiment of the present invention.

### DETAILED DESCRIPTION OF THE INVENTION

The present invention is now described in detail in connection with its various embodiments and with reference to the attached figures.

In a first embodiment, a method for the insertion in a patient of a cochlear implant electrode is provided. Conventionally, the cochlear implant electrode will contain multiple electrode portions. Generally, the cochlear implant electrode will be implanted during a cochlear implantation procedure, which may be conducted through any known surgical approach, but preferably in a minimally invasive approach as will be familiar to those of skill in the art.

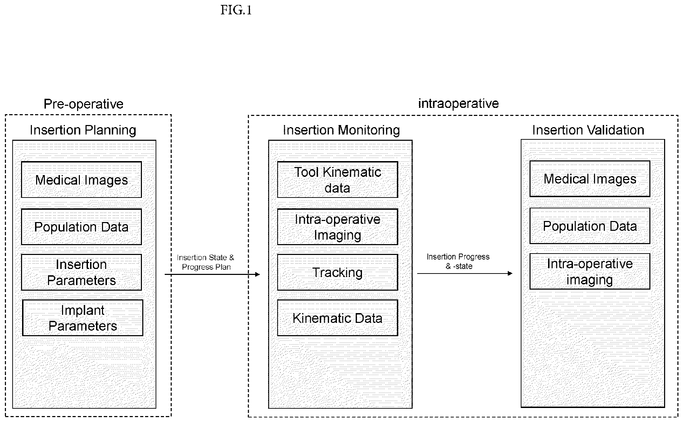

With reference to Figure 1, the method for insertion of a cochlear implant electrode may include the steps of determining a desired insertion state and desired insertion progress plan for the cochlear implant electrode, monitoring insertion progress through available patient, cochlear implant electrode and instrument data and confirming that the cochlear implant electrode has reached the desired insertion state.

A desired insertion state for the cochlear implant electrode is one in which the electrode has reached the desired position in the cochlea of the patient without causing any damage to the cochlea or surrounding tissues and whereby the electrode portions of the cochlear implant electrode are positioned to provide improved hearing performance for the patient.

The medical personnel performing the implantation of the cochlear implant electrode may, prior to the surgical procedure, formulate an insertion progress plan for the insertion of the cochlear implant electrode. The formulation of this plan may include parameters for insertion speed, insertion angle, insertion depth, insertion direction and insertion orientation as measured by conventional means known to one of skill in the art.

During the cochlear implant electrode implantation procedure, insertion progress may be monitored through available patient data, available cochlear implant electrode data and available instrument data. This available information may be compared to the cochlear implant electrode insertion progress plan to continually assess whether the implantation procedure is progressing toward the desired insertion state. Once the procedure data closely matches the desired insertion state, the cochlear implant electrode implantation procedure can be stopped. Carrying out this method by setting a desired insertion state, formulating an insertion progress plan and comparing procedure-obtained data to the insertion progress plan will result in safer implantation procedures for patients and enhanced accuracy in cochlear implant electrode placement.

Still with referenced to Figure 1, determination of the desired insertion state and desired insertion progress plan may be based on patient specific data, cochlear implant electrode specific data and population specific data. Insertion state and progress plan are optimally determined before the implantation procedure by reference to these available sources of data. One of skill in the art will understand that reference to all available sources of data will improve the planning of desired insertion state and insertion progress plan, but reference to less than all available sources of data will still improve accuracy and safety compared to the state of the art which does not include resort to pre-operative planning, determination of desired outcome and monitoring of progress in one implantation method.

The desired insertion state may be expressed in terms of any of cochlear implant electrode entry position, cochlear implant electrode orientation, cochlear implant electrode bending, cochlear implant electrode shape, and number of electrode portions inside the cochlea.

In various embodiments of the present invention, population specific data, patient specific data and cochlear implant electrode specific data may be used in the formulation of a desired insertion state and desired insertion progress plan. Population specific data may include anatomical atlas data, statistical cochlear shape data, and genetic data. Patient specific data may include a given patient's medical image data, audiological data, electrophysiological data and genetic data. Cochlear implant electrode specific data may include a given cochlear implant electrode's physical dimensions, material properties, electrical properties, chemical properties and tribology profile.

In an alternate embodiment of the present invention, the determination of a desired cochlear implant electrode insertion progress plan and desired insertion state includes the selection of an appropriate cochlear implant electrode for the patient. Solely by way of example, if the desired insertion state includes the parameters of cochlear implant electrode entry position, cochlear implant electrode orientation, cochlear implant electrode bending, cochlear implant electrode shape, and number of electrode portions inside the cochlea, each of these parameters will vary based upon patient and population specific data.

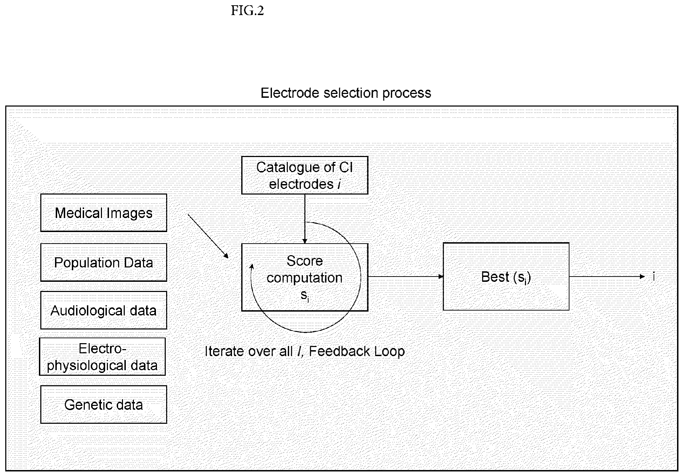

With reference now to Figure 2, and in one embodiment of the present invention, a computerized or automated, iterative process is provided by which patient and population specific data is inputted into a computer with associated software. Based upon the provided data, the computer may select one or more choices of cochlear implant electrodes and generate a score for the electrode choices based upon a desired correlation between the characteristics of the chosen electrode and the inputted data. In an iterative manner, scores may be calculated for each of one or more cochlear implant electrode choices and at the end of the selection process the cochlear implant electrode with the highest score is chosen to be used in the implantation procedure because it is the most likely to produce the desired insertion state and to conform to the desired insertion progress plan.

According to this embodiment of the invention, and in this manner, the ultimate selection of the cochlear implant electrode may be an automated, iterative process using the score computed for each implant choice and employing a computerized planning tool incorporating a database of patient and cochlear implant electrode data.

One of skill in the art will appreciate that, while monitoring insertion progress has been described in the past, it has not been described while taking into account so many different insertion parameters as by the present inventors and it has not been described in conjunction with the formulation, pre-operatively, of a desired insertion state and progress plan informed by an iterative selection process for an optimal cochlear implant electrode for the specific patient.

Accordingly, monitoring insertion progress may be performed using instrument or insertion tool kinematic data, intraoperative imaging, electrocochleography, tissue impedance measurements, stereotactic tracking of the cochlear implant electrode by tracking the cochlear implant electrode or an instrument or insertion tool, measuring insertion force as a function of position and monitoring shape of the cochlear implant electrode. Monitoring the shape of the cochlear implant electrode may be accomplished through intraoperative imaging or by deriving shape from available position and force data. The recited monitoring means will be well known to one skill in the art and can be accomplished through known and available methods.

Similarly, and according to anther embodiment of the present invention, confirming that the cochlear implant electrode has reached the desired insertion state may be achieved through the use of any of instrument or insertion tool kinematic data, intraoperative imaging, electrocochleography, tissue impedance measurements, stereotactic tracking of the cochlear implant electrode or an instrument, measuring insertion force, and visualising or deriving shape of the cochlear implant electrode. As already described herein, visualizing shape can be accomplished by line of sight or by intraoperative imaging and shape can also be derived from available position and force data.

According to alternative embodiments of the present invention, a system for automated insertion of a cochlear implant electrode is provided. The provided system comprises an insertion tool and insertion progress monitoring equipment. The system is configured in its most general sense to provide insertion of a cochlear implant electrode by determining a desired insertion state and desired insertion progress plan for the cochlear implant electrode, monitoring insertion progress through available patient, cochlear implant electrode and instrument data and confirming that the cochlear implant electrode has reached the desired insertion state.

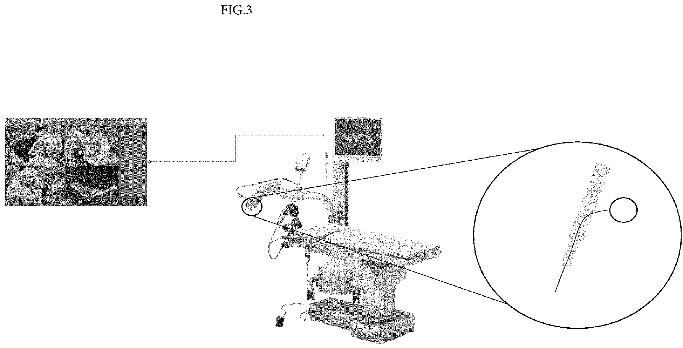

With reference now to Figure 3, the system may include a teleoperated device capable of carrying out kinematic movements with at least three degrees of freedom. This teleoperated device may optionally be a three-axis cartesian robot or a complete surgical robot or any other teleoperated device capable of carrying out the inventive methods. The system may further include a computer that is configured to receive insertion progress data, adjust insertion parameters on the basis of received insertion progress data and determine when an insertion end state has been reached.

According to a further embodiment of the invention, and with reference still to Figure 3, the system comprising a teleoperated device and a computer may further include a computerized planning tool associated with the computer that may include a database of patient, population and cochlear implant electrode data. The computerized planning tool would be configured to receive patient specific data for a specific cochlear implantation procedure and incorporate population specific data and implant specific data to arrive at a desired insertion state and desired insertion progress plan. The system comprising a teleoperated device, a computer, and a computerized planning tool may be configured to allow a feedback or control loop between the computerized planning tool that allows for monitoring of the implantation procedure by comparing insertion state to a desired insertion progress plan and then adjusting surgical parameters to ensure that insertion state conforms to the desired insertion progress plan.

As described herein with reference to earlier embodiments, population specific data may include anatomical atlas data, statistical cochlear shape data, and genetic data, patient specific data may include a given patient's medical image data, audiological data, electrophysiological data and genetic data, and cochlear implant electrode specific data may include a given cochlear implant electrode's physical dimensions, material properties, electrical properties, chemical properties and tribology profile.

According to this embodiment, the system comprising a teleoperated device and a computer that is configured to formulate a desired insertion state and progress plan is also capable of generating and receiving procedural monitoring data and comparing the obtained data to the desired insertion state and progress plan. Monitoring insertion progress may be performed using instrument or insertion tool kinematic data, intraoperative imaging, electrocochleography, tissue impedance measurements, stereotactic tracking of the cochlear implant electrode by tracking the cochlear implant electrode or an instrument or insertion tool, measuring insertion force as a function of position and monitoring shape of the cochlear implant electrode

The system is configured to continuously monitor and receive insertion progress data and create a feedback loop by which comparison of the insertion progress data to the desired insertion state and progress plan provides guidance for continued insertion parameters. Finally, when the desired insertion state is reached, the system will stop the implantation procedure.

While this invention has been shown and described with reference to particular embodiments thereof, it will be understood by those of skill in the art that various changes in form and details may be made therein without departing from the spirit and the scope of the invention as defined by the appended claims. Solely by way of example, not by way of limitation, one of skill in the art will easily understand that the methods/systems disclosed herein can be applied to other surgical fields.

## Claims

1. A method for the insertion of a cochlear implant electrode comprising multiple electrode portions during a cochlear implantation procedure in a patient comprising the steps of:
determining a desired insertion state and desired insertion progress plan for the cochlear implant electrode;
monitoring insertion progress through available patient, cochlear implant electrode and instrument data; and
confirming that the cochlear implant electrode has reached the desired insertion state.

2. The method of claim 1, wherein determining the desired insertion state and desired insertion progress plan is based upon population specific data, patient specific data and cochlear implant electrode specific data and wherein desired insertion progress may be expressed in terms of any of insertion depth, speed, direction, orientation, speed profile and orientation profile and wherein the desired insertion state may be expressed in terms of any of cochlear implant electrode entry position, cochlear implant electrode orientation, cochlear implant electrode bending, cochlear implant electrode shape, and number of electrode portions inside the cochlea.

3. The method of claim 2, wherein population specific data may include anatomical atlas data, statistical cochlear shape data, and genetic data, and wherein patient specific data may include a given patient's medical image data, audiological data, electrophysiological data and genetic data, and wherein cochlear implant electrode specific data may include a given cochlear implant electrode's physical dimensions, material properties, electrical properties, chemical properties and tribology profile.

4. The method of any of the previous claims wherein the step of determining desired insertion states and insertion progress plans includes a preferred cochlear implant electrode choice determined by selecting different cochlear implant electrodes in a sequential manner and in which a computer-generated score is computed for each cochlear implant electrode choice and in which the cochlear implant electrode with the highest score most likely provides an optimal insertion state and insertion progress plan for the given patient.

5. The method of claim 4, wherein an ultimate selection of the cochlear implant electrode may be an automated, iterative process using the score computed for each implant choice and employing a computerized planning tool incorporating a database of patient and cochlear implant electrode data.

6. The method of claim 1, wherein monitoring insertion progress is performed using instrument or insertion tool kinematic data, intraoperative imaging, electrocochleography, tissue impedance measurements, stereotactic tracking of the cochlear implant electrode by tracking the cochlear implant electrode or an instrument or insertion tool, measuring insertion force as a function of position and monitoring shape of the cochlear implant electrode.

7. The method of claim 6, wherein monitoring the shape of the cochlear implant electrode may be performed by intraoperative imaging or by deriving shape from available position and force data.

8. The method of claim 6, comprising the further step of comparing monitored insertion progress data to the desired insertion progress state and desired insertion progress plan to assess whether the desired insertion state may have been reached.

9. The method of claim 1, wherein the step of confirming that the cochlear implant electrode has reached the desired insertion state includes the further step of comparing monitored insertion progress data to the desired insertion state.

10. The method of claim 9, wherein confirming that the cochlear implant electrode has reached the desired insertion state may be achieved through the use of any of instrument or insertion tool kinematic data, intraoperative imaging, electrocochleography, tissue impedance measurements, stereotactic tracking of the cochlear implant electrode or an instrument, measuring insertion force, and visualising or deriving shape of the cochlear implant electrode.

11. A system for automated insertion of a cochlear implant electrode comprising an insertion tool and insertion progress monitoring equipment that is configured to insert a cochlear implant electrode according to the method of any of the previous claims.

12. The system of claim 11 that may further include a computerized planning tool incorporating a database of patient and cochlear implant electrode data and that may be further capable of collecting insertion progress data and comparing the insertion progress data to a preoperative plan.

13. The system of claim 12 wherein the insertion tool is a teleoperated device that is capable of carrying out kinematic movements with at least three degrees of freedom.

14. The system of claim 13, wherein the teleoperated device comprises a computer that is configured to receive insertion progress data, adjust insertion parameters on the basis of received insertion progress data and determine when an insertion end state has been reached.
